# EUROPEAN PATENT SPECIFICATION

(11) **EP 0 951 908 B1**
(45) Date of publication and mention of the grant of the patent: **13.10.2004**
(21) Application number: 99301085.9
(22) Date of filing: 15.02.1999
(51) Int. Cl.: A61K 31/505, A61K 31/535, A61K 31/635

(54) **Treatment of impotence due to spinal cord injury**
Behandlung von Rückenmarkverletzungenbedingter Impotenz
Traitement de l'impuissance due à des léesions de la moelle épinerière

(30) Priority: 23.02.1998 US 75580 P
(43) Date of publication of application: 27.10.1999
(73) Proprietor: Pfizer Limited, Sandwich, Kent CT13 9NJ (GB); Pfizer Ireland Pharmaceuticals, Dun Laoghaire, County Dublin (IE)
(72) Inventor: Maytom, Murray Craig, New York, N.Y. 10017 (US); Osterloh, Ian Howard, Sandwich, Kent CT13 9NJ (GB)
(74) Representative: McMunn, Watson Palmer

(56) References cited:
- WO-A-94/28902
- DERRY, F. A. ET AL: "Efficacy and safety of oral sildenafil (viagra) in men with erectile dysfunction caused by spinal cord injury" NEUROLOGY (1998), 51(6), 1629-1633 , XP000921336
- HOLMGREN, EDDY (1) ET AL: "Sildenafil (VIAGRA) in the treatment of erectile dysfunction (ED) caused by spinal cord injury (SCI): A double-blind, placebo-controlled, flexible-dose, two-way crossover study." NEUROLOGY, (APRIL, 1998) VOL. 50, NO. 4 SUPPL. 4, PP. A127. MEETING INFO.: 50TH ANNUAL MEETING OF THE AMERICAN ACADEMY OF NEUROLOGY MINNEAPOLIS, MINNESOTA, USA APRIL 25-MAY 2, 1998 AMERICAN ACADEMY OF NEUROLOGY. , XP000921335
- DERRY, F. (1) ET AL: "Sildenafil (Viagra): An oral treatment for men with erectile dysfunction caused by traumatic spinal cord injury: A 28-day, double-blind, placebo-controlled, parallel-group, dose-response study." JOURNAL OF THE NEUROLOGICAL SCIENCES, (1997) VOL. 150, NO. SUPPL., PP. S270. MEETING INFO.: XVI WORLD CONGRESS OF NEUROLOGY BUENOS AIRES, ARGENTINA SEPTEMBER 14-19, 1997 , XP000921340
- DERRY, FADEL ET AL: "Sildenafil (Viagra): An oral treatment for men with erectile dysfunction caused by traumatic spinal cord injury - A 28-day, double-blind, placebo-controlled, parallel-group, dose-response study." NEUROLOGY, (1997) VOL. 48, NO. 3 SUPPL. 2, PP. A215. MEETING INFO.: 49TH ANNUAL MEETING OF THE AMERICAN ACADEMY OF NEUROLOGY BOSTON, MASSACHUSETTS, USA APRIL 12-19, 1997 , XP000921334
- DERRY, FADEL (1) ET AL: "Sildenafil (Viagra): A double-blind, placebo-controlled, single-dose, two-way crossover study in men with erectile dysfunction caused by traumatic spinal cord injury." JOURNAL OF UROLOGY, (1997) VOL. 157, NO. 4 SUPPL., PP. 181. MEETING INFO.: 92ND ANNUAL MEETING OF THE AMERICAN UROLOGICAL ASSOCIATION NEW ORLEANS, LOUISIANA, USA APRIL 12-17, 1997 , XP000921337
- DERRY, F. (1) ET AL: "Sildenafil (Viagra): A double-blind, placebo-controlled, single-dose, two-way crossover study in men with erectile dysfunction caused by traumatic spinal cord injury." JOURNAL OF THE NEUROLOGICAL SCIENCES, (1997) VOL. 150, NO. SUPPL., PP. S134. MEETING INFO.: XVI WORLD CONGRESS OF NEUROLOGY BUENOS AIRES, ARGENTINA SEPTEMBER 14-19, 1997 , XP000921341

## Description

### Field Of The Invention

This invention relates to the use of a compound of formula (I) for the manufacture of a medicament for treating sexual dysfunction due to spinal cord injury (SCI) comprising administering an effective amount of said compound of formula I as defined below, including pharmaceutically acceptable salts thereof.

### Background Of The Invention

Impotence is the inability to obtain and/or sustain an erection sufficient for penetration of the vagina and/or intercourse. Thus, impotence is also referred to as "erectile insufficiency" or "erectile dysfunction". It has been estimated that 10-12 million American men between the ages of 18 and 75 suffer from chronic impotence, with the great majority being over age 55.

The penis normally becomes erect when certain tissues, in particular the corpora cavemosa in the central portion of the penis, become engorged with blood, thereby causing them to become rigid, causing an erection. Impotence can result from psychologic disturbances (psychogenic), from physiologic abnormalities (organic) or from a combination of both. Thus, in some males erectile dysfunction may be due to anxiety or depression, with no apparent somatic or organic impairment. In other cases, erectile dysfunction is associated with atherosclerosis of the arteries supplying blood to the penis. In still other cases, the dysfunction may be due to venous leakage or abnormal drainage in which there is leakage from veins in the penis such that sufficient pressure for an erection can be neither obtained nor maintained. In still other cases, the dysfunction is associated with a neuropathy or due to nerve damage arising from, for example, surgery or a pelvic injury. Typically, multiple factors are responsible for impotence.

### Summary Of The Invention

This invention provides the use of a compound for the manufacture of a medicament for treating sexual dysfunction in a female animal with an injured spinal cord, or in a male animal with an injured spinal cord wherein said male animal exhibits no residual erectile function, wherein said compound is a compound of formula (I): 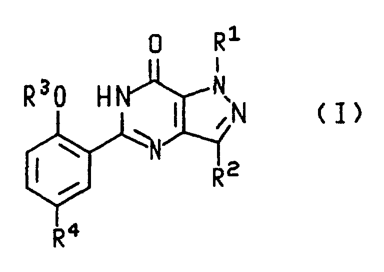 wherein:
R¹ is H; C₁-C₃ alkyl; C₁-C₃ perfluoroalkyl; or C₃-C₅ cycloalkyl;
R² is H; C₁-C₆ alkyl optionally substituted with C₃-C₆ cydoalkyl; C₁-C₃ perfluoroalkyl; or C₃-C₆ cycloalkyl;
R³ is C₁-C₆ alkyl optionally substituted with C₃-C₆ cycloalkyl; C₁-C₆ perfluoroalkyl; C₃-C₅ cycloalkyl; C₃-C₆ alkenyl; or C₃-C₆ alkynyl;
R⁴ is C₁-C₄ alkyl optionally substituted with OH, NR⁵R⁶, CN, CONR⁵R⁶ or CO₂R⁷; C₂-C₄ alkenyl optionally substituted with CN, CONR⁵R⁶ or CO₂R⁷; C₂-C₄ alkanoyl optionally substituted with NR⁵R⁶; (hydroxy)C₂-C₄ alkyl optionally substituted with NR⁵R⁶; (C₂-C₃ alkoxy)C₁-C₂ alkyl optionally substituted with OH or NR⁵R⁶; CONR⁵R⁶; CO₂R⁷; halo; NR⁵R⁶; NHSO₂NR⁵R⁶; NHSO₂R⁸; SO₂NR⁹R¹⁰; or phenyl, pyridyl, pyrimidinyl, imidazolyl, oxazolyl, thiazolyl, thienyl or triazolyl any of which is optionally substituted with methyl;
R⁵ and R⁶ are each independently H or C₁-C₄ alkyl, or together with the nitrogen atom to which they are attached form a pyrrolidinyl, piperidino, morpholino, 4-N(R¹¹)-piperazinyl or imidazolyl group wherein said group is optionally substituted with methyl or OH;
R⁷ is H or C₁-C₄ alkyl;
R⁸ is C₁-C₃ alkyl optionally substituted with NR⁵R⁶;
R⁹ and R¹⁰ together with the nitrogen atom to which they are attached form a pyrrolidinyl, piperidino, morpholino or 4-N(R¹²)-piperazinyl group wherein said group is optionally substituted with C₁-C₄ alkyl, C₁-C₃ alkoxy, NR¹³R¹⁴ or CONR¹³R¹⁴;
R¹¹ is H; C₁-C₃ alkyl optionally substituted with phenyl; (hydroxy)C₂-C₃ alkyl; or C₁-C₄ alkanoyl;
R¹² is H; C₁-C₆ alkyl; (C₁-C₃ alkoxy)C₂-C₆ alkyl; (hydroxy)C₂-C₆ alkyl; (R¹³R¹⁴N)C₂-C₆ alkyl; (R¹³R¹⁴NOC)C₁-C₆ alkyl; CONR¹³R¹⁴; CSNR¹³R¹⁴; or C(NH)NR¹³R¹⁴; and
R¹³ and R¹⁴ are each independently H; C₁-C₄ alkyl; (C₁-C₃ alkoxy)C₂-C₄ alkyl; or (hydroxy)C₂-C₄ alkyl;
or a pharmaceutically acceptable salt thereof;
or a pharmaceutical composition containing either entity.

The above compounds are disclosed, inter alia, in US patents 5,250,534, 5,272,147 and 5,426,107, and in WO 94/28902.

Types of sexual dysfunction due to spinal cord injury which are treatable by means of this invention include male erectile dysfunction and female sexual dysfunction such as orgasmic dysfunction and arousal disorders.

"Sexual dysfunction in an animal with an injured spinal cord" means sexual dysfunction in an animal due to the trauma and/or nerve damage which accompanies a physical spinal cord injury or nerve damage resulting from organic disease. In this type of injury the cortical components of sexual arousal (for example visual sexual stimulation) are disassociated from the localized reflexogenic component of the arousal process. There are, of course, varying degrees of spinal cord injury. The average male patient suffers nerve damage sufficient to prevent the patient from being able to obtain and/or sustain an erection sufficient for intercourse, yet the patient still exhibits a reflexogenic erectile response.

A subset of spinal cord injured patients includes male patients who have no residual erectile function following the injury. Such a patient can be defined as one who exhibits no apparent erectile response, indicating no reflexogenic erectile response to local stimulation, usually penile vibratory stimulation (PVS), and no erections induced by other means (e.g., visual stimulation). It has been determined that use of a compound in accordance with this invention can restore erectile function sufficient for intercourse in a substantial proportion of this SCI patient population. It is truly surprising that erectile function can be restored in a patient who has sustained a SCI to the extent that, in the absence of treatment with a compound of formula (I), local stimulation produces no apparent erectile response.

### Detailed Description

Reference to a compound of formula I, both in this disclosure and the appendant claims, shall at all times be understood to include all active forms of such compounds, including the free form thereof (e.g., the free acid or base form) and also all pharmaceutically acceptable salts, prodrugs, polymorphs, hydrates, solvates, stereoisomers (e.g. diastereomers and enantiomers), and so forth. Active metabolites of such compounds are also included.

Preferred compounds of formula (I) include those which can be taken as required, as compared with needing to be taken chronically. Such preferred compounds include those which improve the sexual response such that the patient responds to sexual (e.g., visual and/or tactile) stimulation, as opposed to compounds which act by causing an erection in the absence of sexual stimulation.

Additional preferred compounds include those which are "fast acting", meaning that the time taken from administration to the point at which the sexual response is improved is less than about two hours, preferably less than about one hour, more preferably on the order of a half hour or less, and even more preferably within 10 or 15 minutes.

Preferred compounds (which are cGMP PDEᵥ inhibitors) include sildenafil, 5-[2-ethoxy-5-(4-methyl-1-piperazinylsulphonyl)-phenyl]-1-methyl-3-n-propyl-1,6-dihydro-7H-pyrazolo[4,3-d]pyrimidin-7-one, which has the structure: 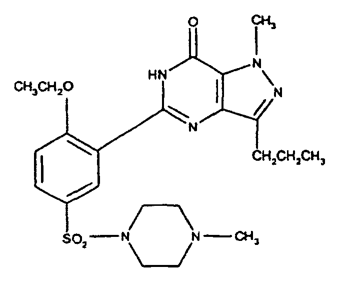 and pharmaceutically acceptable salts thereof, and the compound having the structure: 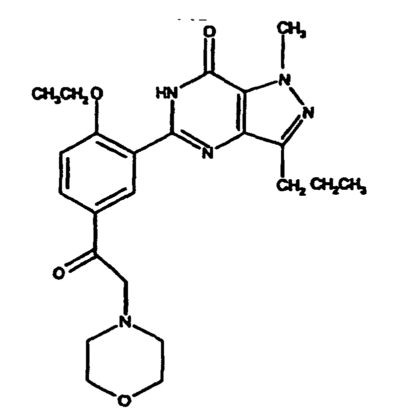 and pharmaceutically acceptable salts thereof. The first compound, sildenafil, is disclosed in US patent 5,250,534. The second compound is disclosed, for example, in US patents 5,272,147 and 5,426,107.

A preferred pharmaceutically acceptable salt of sildenafil for use in this invention is the citrate salt, disclosed in co-pending U. S. provisional Apprication No. 60/027,690 filed October 8, 1996.

Other preferred compounds of formula (I) include those compounds selected from:
5-(5-morpholinoacetyl-2-n-propoxyphenyl)-1-methyl-3-n-propyl-1,6-dihydro-7H-pyrazolo[4,3-d]pyrimidin-7-one;
5-[2-allyloxy-5-(4-methyl-1-piperazinylsulphonyl)-phenyl]-1-methyl-3-n-propyl-1,6-dihydro-7H-pyrazolo[4,3-d]pyrimidin-7-one;
5-{2-ethoxy-5-[4-(2-propyl)-1-piperazinyl-sulphonyl]phenyl}-1-methyl-3-n-propyl-1,6-dihydro-7H-pyrazolo[4,3-d]pyrimidin-7-one;
5-{2-ethoxy-5-[4-(2-hydroxyethyl)-1-piperazinyl-sulphonyl]phenyl}-1-methyl-3-n-propyl-1,6-dihydro-7H-pyrazolo[4,3-d]pyrimidin-7-one;
5-{5-[4-(2-hydroxyethyl)-1-piperazinylsulphonyl]-2-n-propoxyphenyl}-1-methyl-3-n-propyl-1,6-dihydro-7H-pyrazolo[4,3-d]pyrimidin-7-one;
5-[2-ethoxy-5-(4-methyl-1-piperazinylcarbonyl)-phenyl]-1-methyl-3-n-propyl-1,6-dihydro-7H-pyrazolo[4,3-d]pyrimidin-7-one; and
5-[2-ethoxy-5-(1-methyl-2-imidazolyl)phenyl]-1-methyl-3-n-propyl-1,6-dihydro-7H-pyrazolo[4,3-d]pyrimidin-7-one.

The above compounds are disclosed in the aforementioned US patents 5,250,534, 5,272,147 and 5,426,107.

A compound of formula I will generally be administered via any of the known routes of administration such as oral, parenteral via local injection intracavemosally or intraurethrally, or transdermal as by applying the active component in a gel or other such formulation topically to the penis. Oral administration is preferred. The compound can be formulated as known in the art, usually together with a pharmaceutically acceptable carrier or diluent, for example as a tablet, capsule, lozenge, troche, elixir, solution, or suspension for oral administration, in a suitable injectable vehicle for parenteral administration, or as a lotion, ointment or cream for topical application.

The exact dose administered will, of course, differ depending on the specific compound of formula I prescribed, on the subject being treated, on the severity of the organic dysfunction, on the manner of administration and on the judgment of the prescribing physician. Thus, because of patient-to-patient variability, the dosages given below are a guideline and the physician may adjust doses of the compounds to achieve the treatment that the physician considers appropriate for the patient. In considering the degree of treatment desired, the physician must balance a variety of factors such as the age and sex of the patient and the presence of other diseases or conditions (e.g., cardiovascular disease). In general, the compound of formula I will be administered in a range of from 10 to 200 mg, preferably 25 to 100 mg, taken as required not more than once daily. Usually, the compound will be taken on demand, anywhere from a few minutes up to several hours prior to intercourse. As previously noted, a compound of formula I can be administered in any conventional oral, parenteral, rectal or transdermal dosage form, usually also together with a pharmaceutically acceptable carrier or diluent.

For oral administration a pharmaceutical composition can take the form of solutions, suspensions, tablets, pills, capsules, powders, and the like. Tablets containing various excipients such as sodium citrate, calcium carbonate and calcium phosphate are employed along with various disintegrants such as starch and preferably potato or tapioca starch and certain complex silicates, together with binding agents such as polyvinylpyrrolidone, sucrose, gelatin and acacia. Additionally, lubricating agents such as magnesium stearate, sodium lauryl sulfate and talc are often very useful for tabletting purposes. Solid compositions of a similar type are also employed as fillers in soft and hard-filled gelatin capsules; preferred materials in this connection also include lactose or milk sugar as well as high molecular weight polyethylene glycols. When aqueous suspensions and/or elixirs are desired for oral administration, a compound of formula I can be combined with various sweetening agents, flavoring agents, coloring agents, emulsifying agents and/or suspending agents, as well as such diluents as water, ethanol, propylene glycol, glycerin and various like combinations thereof.

For purposes of parenteral administration, solutions in sesame or peanut oil or in aqueous propylene glycol can be employed, as well as sterile aqueous solutions of the corresponding water-soluble salts. Such aqueous solutions may be suitably buffered, if necessary, and the liquid diluent first rendered isotonic with sufficient saline or glucose. These aqueous solutions are especially suitable for intravenous, intramuscular, subcutaneous and intraperitoneal injection purposes. In this connection, the sterile aqueous media employed are all readily obtainable by standard techniques well-known to those skilled in the art.

For purposes of transdermal (e.g.,topical) administration, dilute sterile, aqueous or partially aqueous solutions (usually in about 0.1% to 5% concentration), otherwise similar to the above parenteral solutions, are prepared.

Methods of preparing various pharmaceutical compositions with a certain amount of active ingredient are known, or will be apparent in light of this disclosure, to those skilled in this art. For examples of methods of preparing pharmaceutical compositions, see Remington's Pharmaceutical Sciences, Mack Publishing Company, Easter, Pa., 15th Edition (1975).

## Claims

1. . The use of a compound for the manufacture of a medicament for treating sexual dysfunction in a female animal with an injured spinal cord, or in a male animal with an injured spinal cord wherein said male animal exhibits no residual erectile function, wherein said compound is a compound of formula (I): 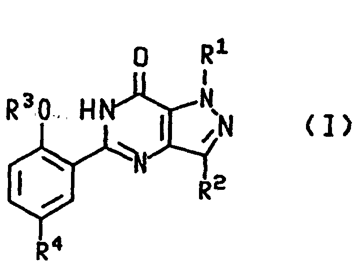 wherein:
R¹ is H; C₁-C₃ alkyl; C₁-C₃ perfluoroalkyl; or C₃-C₅ cycloalkyl;
R² is H; C₁-C₆ alkyl optionally substituted with C₃-C₆ cycloalkyl; C₁-C₃ perfluoroalkyl; or C₃-C₆ cycloalkyl;
R³ is C₁-C₆ alkyl optionally substituted with C₃-C₆ cycloalkyl; C₁-C₆ perfluoroalkyl; C₃-C₅ cycloalkyl; C₃-C₆ alkenyl; or C₃-C₆ alkynyl;
R⁴ is C₁-C₄ alkyl optionally substituted with OH, NR⁵R⁶, CN, CONR⁵R⁶ or CO₂R⁷; C₂-C₄ alkenyl optionally substituted with CN, CONR⁵R⁶ or CO₂R⁷; C₂-C₄ alkanoyl optionally substituted with NR⁵R⁶; (hydroxy)C₂-C₄ alkyl optionally substituted with NR⁵R⁶; (C₂-C₃ alkoxy)C₁-C₂ alkyl optionally substituted with OH or NR⁵R⁶; CONR⁵R⁶; CO₂R⁷; halo; NR⁵R⁶; NHSO₂NR⁵R⁶; NHSO₂R⁸; SO₂NR⁹R¹⁰; or phenyl, pyridyl, pyrimidinyl, imidazolyl, oxazolyl, thiazolyl, thienyl or triazolyl any of which is optionally substituted with methyl;
R⁵ and R⁶ are each independently H or C₁-C₄ alkyl, or together with the nitrogen atom to which they are attached form a pyrrolidinyl, piperidino, morpholino, 4-N(R¹¹)-piperazinyl or imidazolyl group wherein said group is optionally substituted with methyl or OH;
R⁷ is H or C₁-C₄ alkyl;
R⁸ is C₁-C₃ alkyl optionally substituted with NR⁵R⁶;
R⁹ and R¹⁰ together with the nitrogen atom to which they are attached form a pyrrolidinyl, piperidino, morpholino or 4-N(R¹²)-piperazinyl group wherein said group is optionally substituted with C₁-C₄ alkyl, C₁-C₃ alkoxy, NR¹³R¹⁴ or CONR¹³R¹⁴;
R¹¹ is H; C₁-C₃ alkyl optionally substituted with phenyl; (hydroxy)C₂-C₃ alkyl; or C₁-C₄ alkanoyl;
R¹² is H; C₁-C₆ alkyl; (C₁-C₃ alkoxy)C₂-C₆ alkyl; (hydroxy)C₂-C₆ alkyl; (R¹³R¹⁴N)C₂-C₆ alkyl; (R¹³R¹⁴NOC)C₁-C₆ alkyl; CONR¹³R¹⁴; CSNR¹³R¹⁴; or C(NH)NR¹³R¹⁴; and
R¹³ and R¹⁴ are each independently H; C₁-C₄ alkyl; (C₁-C₃ alkoxy)C₂-C₄ alkyl; or (hydroxy)C₂-C₄ alkyl;
or a pharmaceutically acceptable salt thereof;
or a pharmaceutical composition containing either entity.

2. A use as defined in claim 1, wherein said compound is selected from sildenafil, and pharmaceutically acceptable salts thereof, and the compound having the structure: 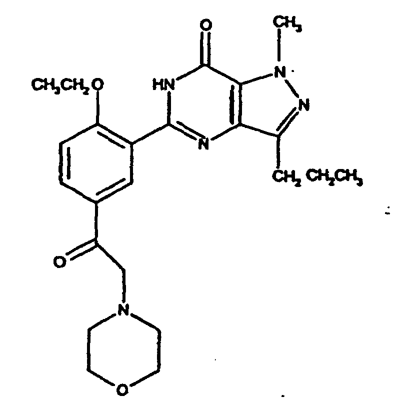 and pharmaceutically acceptable salts thereof.

3. A use as defined in claim 1, wherein said compound is sildenafil or a pharmaceutically acceptable salt thereof.

4. A use as defined in claim 3, wherein said pharmaceutically acceptable salt is the citrate.

5. Use according to any one of the preceding claims wherein the treatment is in a female human and the female sexual dysfunction due to spinal cord injury is orgasmic dysfunction and arousal disorders.

6. Use according to any one of the preceding claims 1 to 4 wherein said male animal is a human male.

7. Use according to any one of the preceding claims wherein the treatment is by oral administration.

8. Use according to any one of the preceding claims wherein the compound of formula I is administered in a range of 10 to 200mg.

9. Use according to claim 8 wherein the compound is administered in a range of 25 to 100mg.

## Patentansprüche

1. Verwendung einer Verbindung für die Herstellung eines Arzneimittels zur Behandlung sexueller Dysfunktion bei einem weiblichen Lebewesen mit einer verletzten Wirbelsäule oder bei einem männlichen Lebewesen mit einer verletzten Wirbelsäule, wobei der Mann bzw. das männliche Tier keine erektile Restfunktion zeigt, wobei die Verbindung eine Verbindung der Formel I 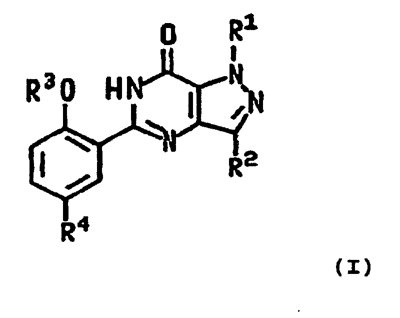 worin
R¹ H, C₁-C₃-Alkyl, C₁-C₃-Perfluoralkyl oder C₃-C₅-Cycloalkyl ist,
R² H; mit C₃-C₆-Cycloalkyl gegebenenfalls substituiertes C₁-C₆-Alkyl, C₁-C₃-Perfluoralkyl oder C₃-C₆-Cycloalkyl ist,
R³ gegebenenfalls mit C₃-C₆-Cycloalkyl substituiertes C₁-C₆-Alkyl; C₁-C₆-Perfluoralkyl; C₃-C₅-Cycloalkyl; C₃-C₆-Alkenyl
oder C₃-C₆-Alkinyl ist,
R⁴ gegebenenfalls mit OH, NR⁵R⁶, CN, CONR⁵R⁶ oder CO₂R⁷ substituiertes C₁-C₄-Alkyl; mit CN, CONR⁵R⁶ oder CO₂R⁷ gegebenenfalls substituiertes C₂-C₄-Alkenyl; gegebenenfalls mit NR⁵R⁶ substituiertes C₂-C₄-Alkanoyl; gegebenenfalls mit NR⁵R⁶ substituiertes (Hydroxy)C₂-C₄alkyl; gegebenenfalls mit OH oder NR⁵R⁶ substituiertes (C₂-C₃-Alkoxy)C₁-C₂-alkyl; CONR⁵R⁶; CO₂R⁷; Halogen; NR⁵R⁶; NHSO₂NR⁵R⁶; NHSO₂R⁸; SO₂NR⁹R¹⁰; oder Phenyl, Pyridyl, Pyrimidinyl, Imidazolyl, Oxazolyl, Thiazolyl, Thienyl oder Triazolyl ist, wobei diese gegebenenfalls mit Methyl substituiert sein können,
R⁵ und R⁶ jeweils unabhängig voneinander für H oder C₁-C₄-Alkyl stehen oder zusammen mit dem Stickstoffatom, an das sie gebunden sind, eine Pyrrolidinyl-, Piperidin-, Morpholin-, 4-N(R¹¹)-Piperazinyl oder Imidazolyl-Gruppe bilden, wobei die Gruppe gegebenenfalls mit Methyl oder OH substituiert ist,
R⁷ H oder C₁-C₄-Alkyl ist;
R⁸ gegebenenfalls mit NR⁵R⁶ substituiertes C₁-C₃-Alkyl ist; R⁹ und R¹⁰ zusammen mit dem Stickstoffatom, an das sie gebunden sind, eine Pyrrolidinyl-, Piperidin-, Morpholinoder 4-N(R¹²)-Piperazinyl-Gruppe bilden, wobei die Gruppe gegebenenfalls mit C₁-C₄-Alkyl, C₁-C₃-Alkoxy, NR¹³R¹⁴ oder CONR¹³R¹⁴ substituiert ist;
R¹¹ H; gegebenenfalls mit Phenyl substituiertes C₁-C₃-Alkyl, (Hydroxy)C₂-C₃-alkyl; oder C₁-C₄-Alkanoyl ist;
R¹² H; C₁-C₆-Alkyl; (C₁-C₃-Alkoxy)C₂-C₆-alkyl; (Hydroxy)C₂-C₆-alkyl; (R¹³R¹⁴N)C₂-C₆-Alkyl; (R¹³R¹⁴NOC)C₁-C₆-Alkyl; CONR¹³R¹⁴; CSNR¹³R¹⁴ oder C(NH)NR¹³R¹⁴ ist; und
R¹³ und R¹⁴ jeweils unabhängig voneinander H; C₁-C₄-Alkyl, (C₁-C₃-Alkoxy)C₂-C₄-alkyl; oder (Hydroxy)C₂-C₄-alkyl sind;
oder ein pharmazeutisch annehmbares Salz davon;
oder eine pharmazeutische Zusammensetzung, die diese enthält, ist.

2. Verwendung nach Anspruch 1, worin die Verbindung aus Sildenafil und pharmazeutisch annehmbaren Salzen davon und der Verbindung, die die Struktur: 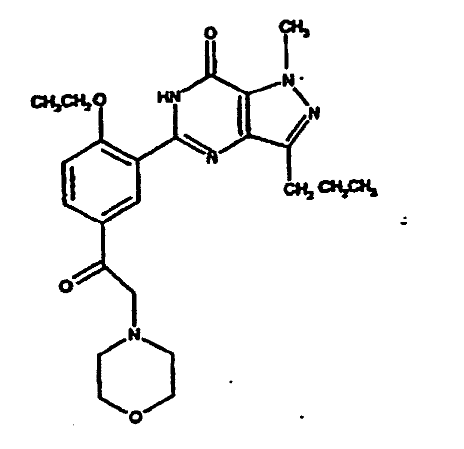 aufweist, und pharmazeutisch annehmbaren Salzen davon ausgewählt ist.

3. Verwendung nach Anspruch 1, worin die Verbindung Sildenafil oder ein pharmazeutisch annehmbares Salz davon ist.

4. Verwendung nach Anspruch 3, worin das pharmazeutisch annehmbare Salz das Citrat ist.

5. Verwendung nach einem der vorhergehenden Ansprüche, worin die Behandlung bei einer Frau erfolgt und die weibliche sexuelle Dysfunktion infolge einer Wirbelsäulenverletzung orgasmische Dysfunktion und Erregungsstörungen ist.

6. Verwendung nach einem der vorhergehenden Ansprüche 1 bis 4, worin das männliche Lebewesen ein Mann ist.

7. Verwendung nach einem der vorhergehenden Ansprüche, worin die Behandlung durch orale Verabreichung erfolgt.

8. Verwendung nach einem der vorhergehenden Ansprüche, worin die Verbindung der Formel I in einem Bereich von 10 bis 200 mg verabreicht wird.

9. Verwendung nach Anspruch 8, worin die Verbindung in einem Bereich von 25 bis 100 mg verabreicht wird.

## Revendications

1. Utilisation d'un composé pour la fabrication d'un médicament destiné à traiter un dysfonctionnement sexuel chez un animal femelle avec une moelle épinière lésée, ou chez un animal mâle avec une moelle épinière lésée où ledit animal mâle ne présente aucune fonction érectile résiduelle, dans laquelle ledit composé est un composé de formule (I) : 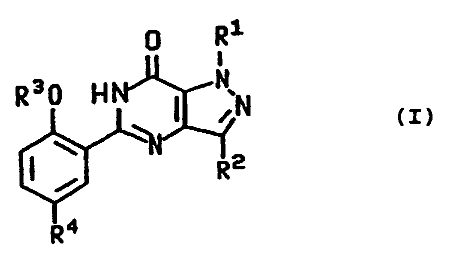 dans laquelle :
R¹ représente un atome de H ; un groupe alkyle en C₁ à C₃ ; un groupe perfluoroalkyle en C₁ à C₃ ; ou un groupe cycloalkyle en C₃ à C₅ ;
R² représente un atome de H, un groupe alkyle en C₁ à C₆ éventuellement substitué par un groupe cycloalkyle en C₃ à C₆ ; un groupe perfluoroalkyle en C₁ à C₃ ; ou un groupe cycloalkyle en C₃ à C₆ ;
R³ représente un groupe alkyle en C₁ à C₆ éventuellement substitué par un groupe cycloalkyle en C₃ à C₆ ; un groupe perfluoroalkyle en C₁ à C₆ ; un groupe cycloalkyle en C₃ à C₅ ; un groupe alcényle en C₃ à C₆ ; ou un groupe alcynyle en C₃ à C₆ ;
R⁴ représente un groupe alkyle en C₁ à C₄ éventuellement substitué par OH, NR⁵R⁶, CN, CONR⁵R⁶ ou CO₂R⁷ ; un groupe alcényle éventuellement substitué par CN, CONR⁵R⁶ ou CO₂R⁷ ; un groupe alcanoyle en C₂ à C₄ éventuellement substitué par NR⁵R⁶ ; un groupe (hydroxy)-alkyle en C₂ à C₄ éventuellement substitué par NR⁵R⁶ ; un groupe (alcoxy en C₂ à C₃)-alkyle en C₁ à C₂ éventuellement substitué par OH ou NR⁵R⁶ ; CONR⁵R⁶ ; CO₂R⁷ ; un groupe halogéno ; NR⁵R⁶ ; NHSO₂NR⁵R⁶ ; NHSO₂R⁸ ; SO₂NR⁹R¹⁰ ; ou un groupe phényle, pyridyle, pyrimidinyle, imidazolyle, oxazolyle, thiazolyle, thiényle ou triazolyle dont l'un quelconque est éventuellement substitué par un groupe méthyle ;
R⁵ et R⁶ représentent chacun, indépendamment l'un de l'autre, un atome de H ou un groupe alkyle en C₁ à C₄, ou conjointement avec l'atome d'azote auquel ils sont attachés forment un groupe pyrrolidinyle, pipéridino, morpholino, 4-N(R¹¹)-pipérazinyle ou imidazolyle dans lequel ledit groupe est éventuellement substitué par un groupe méthyle ou OH ;
R⁷ représente un atome de H ou un groupe alkyle en C₁ à C₄ ;
R⁸ représente un groupe alkyle en C₁ à C₃ éventuellement substitué par NR⁵R⁶ ;
R⁹ et R¹⁰ conjointement avec l'atome d'azote auquel ils sont attachés forment un groupe pyrrolidinyle, pipéridino, morpholino ou 4-N(R¹²)-pipérazinyle dans lequel ledit groupe est éventuellement substitué par un groupe alkyle en C₁ à C₄, alcoxy en C₁ à C₃, NR¹³R¹⁴ ou CONR¹³R¹⁴ ;
R¹¹ représente un atome de H ; un groupe alkyle en C₁ à C₃ éventuellement substitué par un groupe phényle ; un groupe (hydroxy)-alkyle en C₂ à C₃ ; ou un groupe alcanoyle en C₁ à C₄ ;
R¹² représente un atome de H ; un groupe alkyle en C₁ à C₆ ; un groupe (alcoxy en C₁ à C₃)-alkyle en C₂ à C₆ ; un groupe (hydroxy)-alkyle en C₂ à C₆ ; un groupe (R¹³R¹⁴N)-alkyle en C₂ à C₆ ; un groupe (R¹³R¹⁴NOC)-alkyle en C₁ à C₆ ; CONR¹³R¹⁴ ; CSNR¹³R¹⁴ ; ou C(NH)NR¹³R¹⁴ ; et
R¹³ et R¹⁴ représentent chacun, indépendamment l'un de l'autre, un atome de H ; un groupe alkyle en C₁ à C₄ ; un groupe (alcoxy en C₁ à C₃)-alkyle en C₂ à C₄ ; ou un groupe (hydroxy)-alkyle en C₂ à C₄ ;
ou un de ses sels pharmaceutiquement acceptable ;
ou une composition pharmaceutique contenant chaque entité.

2. Utilisation selon la revendication 1, dans laquelle ledit composé est choisi parmi le sildénafil, et un de ses sels pharmaceutiquement acceptable, et le composé possédant la structure : 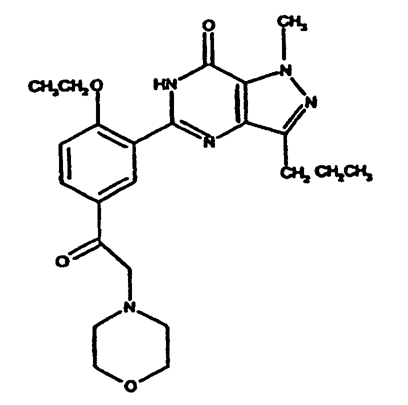 et ses sels pharmaceutiquement acceptables.

3. Utilisation selon la revendication 1, dans laquelle ledit composé est le sildénafil ou un de ses sels pharmaceutiquement acceptable.

4. Utilisation selon la revendication 3, dans laquelle ledit sel pharmaceutiquement acceptable est le citrate.

5. Utilisation selon l'une quelconque des revendications précédentes, dans laquelle le traitement se fait chez un humain de sexe féminin et le dysfonctionnement sexuel féminin dû à des lésions de la moelle épinière est un dysfonctionnement orgastique et des troubles de l'excitation.

6. Utilisation selon l'une quelconque des revendications 1 à 4 précédentes, dans laquelle ledit animal mâle est un humain de sexe masculin.

7. Utilisation selon l'une quelconque des revendications précédentes, dans laquelle le traitement se fait par administration orale.

8. Utilisation selon l'une quelconque des revendications précédentes, dans laquelle on administre le composé de formule I dans une gamme de 10 à 200 mg.

9. Utilisation selon la revendication 8, dans laquelle on administre le composé dans une gamme de 25 à 100 mg.
